(19) Europäisches Patentamt
European Patent Office
Office européen des brevets

(11) Publication number: **0 155 094 A1**

# (12) EUROPEAN PATENT APPLICATION

(21) Application number: **85301114.6**

(22) Date of filing: **20.02.85**

(51) Int. Cl.⁴: **C 07 D 471/04**, A 61 K 31/435 // (C07D471/04, 235:00, 221:00)

(30) Priority: **24.02.84 US 583518**

(43) Date of publication of application: **18.09.85 Bulletin 85/38**

(84) Designated Contracting States: **AT BE CH DE FR GB IT LI LU NL SE**

(71) Applicant: **ELI LILLY AND COMPANY, Lilly Corporate Center, Indianapolis Indiana 46285 (US)**

(72) Inventor: **Robertson, David Wayne, 3712 Ivory Way East, Indianapolis Indiana 46227 (US)**

(74) Representative: **Tapping, Kenneth George et al, Lilly Industries Limited Patent Department Erl Wood Manor, Windlesham Surrey, GU20 6PH (GB)**

## (54) 2-Arylimidazo(4,5-c)pyridines.

(57) 2-Arylimidazo[4,5-c]pyridines having the formulae

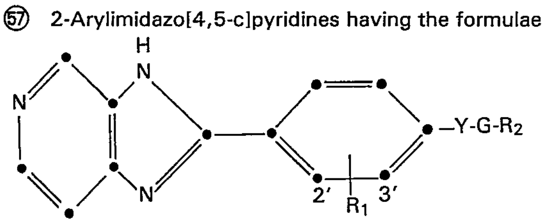

I

and

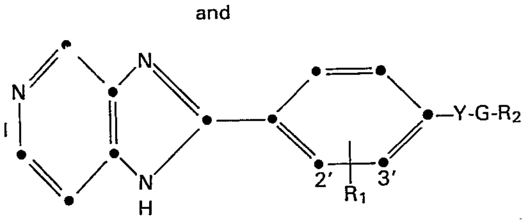

Ia

or a pharmaceutically acceptable salt thereof, wherein:

$R_1$ is hydrogen, $C_1$-$C_4$ alkyl, $C_1$-$C_4$ alkoxy, allyloxy, propargyloxy, benzyloxy, ($C_1$-$C_4$ alkyl)thio, ($C_1$-$C_4$ alkyl)sulfinyl, ($C_1$-$C_4$ alkyl)sulfonyl, hydroxy, halo, cyano, nitro, amino, mono- or di-($C_1$-$C_4$ alkyl)amino, trifluoromethyl, or Z-Q-substituted $C_1$-$C_4$ alkoxy, wherein Q is oxygen, sulfur, sulfinyl, sulfonyl, or a bond, and Z is $C_1$-$C_4$ alkyl, phenyl or phenyl substituted with halo, $C_1$-$C_4$ alkyl, $C_1$-$C_4$ alkoxy, hydroxy, nitro, amino, ($C_1$-$C_4$ alkyl)thio, ($C_1$-$C_4$ alkyl)sulfinyl, or ($C_1$-$C_4$ alkyl)-sulfonyl;

Y is -O- or -NH-;

G is C=O or $-S(O)_r-$, where r is 0, 1, or 2;

and

$R_2$ is $C_1$-$C_4$ alkyl, phenyl, trifluoromethyl, or $-NR_3R_4$, where each of $R_3$ and $R_4$ is independently hydrogen or $C_1$-$C_4$ alkyl; with the limitations that when $R_2$ is trifluoromethyl or $-NR_3R_4$, and G is $-S(O)_r-$, r may not be 0 or 1, and with the further limitation that when Y is -O- and G is $-S(O)_r-$, r may not be 0 or 1.

The compounds are useful for treating a mammal, including a human, suffering from or susceptible to the conditions of asthma, thrombosis, hypertension, or heart failure.

EP 0 155 094 A1

# 2-ARYLIMIDAZO[4,5-c]PYRIDINES

This invention provides long-acting orally effective positive inotropic agents.

The cardiac glycosides and the sympathomimetic amines are the principal inotropic agents used in the management of congestive heart failure. Although the cardiac glycosides, especially digitalis, are among the most frequently prescribed drugs, they have numerous liabilities such as a low therapeutic index and erratic absorption, and are associated with life-threatening arrhythmias and deleterious drug-drug interactions. In addition, many patients either do not respond, or become refractory to these agents. The sympathomimetic amines, such as dopamine and epinephrine, have limited utility due to positive chronotropic effects, arrhythmogenic properties, and oral ineffectiveness.

More recently, new classes of inotropic agents have been found. Among these, certain 2-phenylimidazo-[4,5-b]pyridines (U.S. Patents No. 3,985,891 and 4,327,100) have been shown to possess inotropic and anticoagulant activity. U.S. Patents No. 4,299,834 and 4,353,909 describe similarly substituted purine and 6-hydroxy-imidazo[4,5-b]pyridine derivatives, respectively. The analogous imidazo[4,5-c]pyridines have also been taught to possess inotropic activity; see European Patent Applications 72,926 and 79,083 and British Patent Application No. 2,119,377.

The present invention provides for a series of imidazo derivatives, their pharmaceutical formulations, and their use as long-acting orally effective positive inotropic agents which have minimal effects on blood pressure and heart rate. The compounds also possess vasodilatory, bronchodilatory, and anticoagulant activities.

This invention provides for pharmaceutically useful aryl-substituted imidazo compounds having the formulae

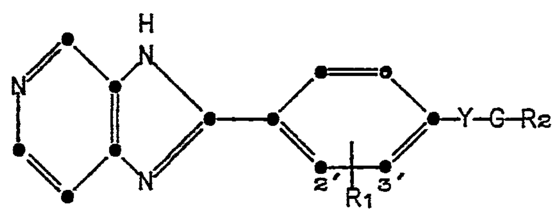

I

and

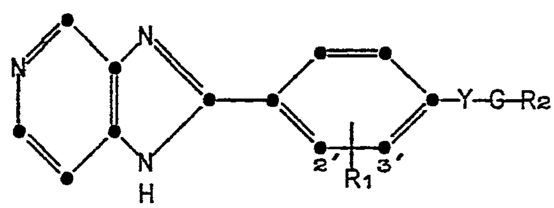

Ia

or a pharmaceutically acceptable salt thereof, wherein:

$R_1$ is hydrogen, $C_1$-$C_4$ alkyl, $C_1$-$C_4$ alkoxy, allyloxy, propargyloxy, benzyloxy, ($C_1$-$C_4$ alkyl)thio, ($C_1$-$C_4$ alkyl)sulfinyl, ($C_1$-$C_4$ alkyl)sulfonyl, hydroxy, halo, cyano, nitro, amino, mono- or di-($C_1$-$C_4$ alkyl)-amino, trifluoromethyl, or Z-Q-substituted $C_1$-$C_4$ alkoxy,

wherein Q is oxygen, sulfur, sulfinyl, sulfonyl, or a bond, and Z is $C_1$-$C_4$ alkyl, phenyl or phenyl substituted with halo, $C_1$-$C_4$ alkyl, $C_1$-$C_4$ alkoxy, hydroxy, nitro, amino, ($C_1$-$C_4$ alkyl)thio, ($C_1$-$C_4$ alkyl)sulfinyl, or ($C_1$-$C_4$ alkyl)sulfonyl;

Y is -O- or -NH-;

G is C=O or $-S(O)_r-$, where r is 0, 1, or 2; and

$R_2$ is $C_1$-$C_4$ alkyl, phenyl, trifluoromethyl, or $-NR_3R_4$,

where each of $R_3$ and $R_4$ is independently hydrogen or $C_1$-$C_3$ alkyl; with the limitations that when $R_2$ is trifluoromethyl or $-NR_3R_4$, and G is $-S(O)_r-$, r may not be 0 or 1, and with the further limitation that when Y is -O- and G is $-S(O)_r-$, r may not be 0 or 1.

The compounds are useful for treating a mammal, including a human, suffering from or susceptible to the conditions of asthma, thrombosis, hypertension, or heart failure.

According to a further aspect of the present invention there is provided a pharmaceutical formulation which comprises as active ingredient a compound of Formula I or Ia as defined above, associated with a pharmaceutically acceptable carrier.

The Formulae I and Ia are recognized as being tautomeric structures of one another. The imidazo compounds having the hydrogen atom on one of the imidazo nitrogen atoms (e.g., Formula I) have corresponding tautomeric forms wherein the hydrogen atom is on the other imidazo nitrogen atom (e.g., Formula Ia). As N-unsubstituted compounds, each tautomeric form exists

in equilibrium with the other and cannot be prepared or isolated without the presence of the other. For this application, both forms will be considered together. Thus, the compounds of Formula I (Ia) are known as 2-(aryl)-1H(or 3H)-imidazo[4,5-c]pyridines. For simplicity, these compounds will be referred to as compounds of Formula I (Ia) or by name without designating on which nitrogen atom the hydrogen atom is found.

A preferred group of compounds are the compounds of Formula I (Ia) wherein $R_1$ is hydrogen, $C_1$-$C_4$ alkyl, $C_1$-$C_4$ alkoxy, halogen, ($C_1$-$C_4$ alkyl)thio, ($C_1$-$C_4$ alkyl)sulfinyl, ($C_1$-$C_4$ alkyl)sulfonyl, or Z-Q-substituted $C_1$-$C_4$ alkoxy; and pharmaceutically acceptable salts thereof.

Especially preferred compounds as defined above are those where "$C_1$-$C_4$ alkyl" is methyl, "($C_1$-$C_4$ alkyl)sulfinyl" is methyl sulfinyl, "($C_1$-$C_4$ alkyl)-sulfonyl" is methyl sulfonyl, and "$C_1$-$C_4$ alkoxy" is methoxy. Preferred Z-Q-substituted $C_1$-$C_4$ alkoxy compounds are those wherein $C_1$-$C_4$ alkoxy is ethoxy or n-propoxy, Q is oxygen, sulfur or sulfinyl, and Z is $C_1$-$C_4$ alkyl, phenyl or phenyl substituted with halo, $C_1$-$C_4$ alkoxy, or hydroxy. Compounds wherein $R_1$ is hydrogen or $C_1$-$C_4$ alkoxy, especially methoxy, are particularly preferred. It is preferred that $R_1$ is attached at the 2'-position of the phenyl ring. Also preferred are those compounds wherein G is $-S(O)_r-$, especially where r is 2.

The following definitions refer to the various terms used throughout this disclosure.

The term "halo" refers to fluoro, chloro, bromo, and iodo.

The term "$C_1$-$C_4$ alkyl" refers to the straight and branched aliphatic radicals of one to four carbon atoms including methyl, ethyl, propyl, isopropyl, butyl, isobutyl, sec-butyl, and tert-butyl, and includes within it the term "$C_1$-$C_3$ alkyl."

The term "$C_1$-$C_4$ alkoxy" includes the straight and branched aliphatic ether radicals of one to four carbon atoms such as methoxy, ethoxy, propoxy, isopropoxy, butoxy, isobutoxy, sec-butoxy, and tert-butoxy.

The compounds of this invention as represented by Formulas I and Ia may be prepared by any of several methods known in the art.

A preferred method of preparation consists of the reaction of an amine of the formula

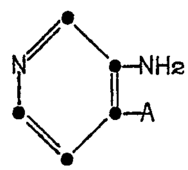

II

wherein A is amino, with an aryl derivative of the formula

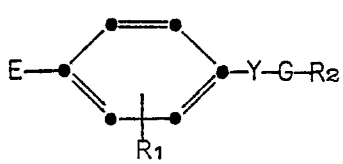

III

wherein E is -COOH. The reaction may be performed in the absence of a solvent, but is generally carried out in a suitable non-reactive solvent, such as benzene, toluene, xylene, ethylene glycol, pyridine, acetone, phosphorous oxychloride, polyphosphoric acid, and the like, optionally in the presence of a base, a catalytic amount of an acid, or a dehydrating agent. Preferred bases are pyridine or triethylamine, p-toluenesulfonic is the preferred acid, and dehydrating agents, such as phosphorous oxychloride, phosphorous pentoxide, or thionyl chloride may be used. Temperatures in the range of -20°C. to 250°C. may be employed with a preferred range of 50-200°C.

Other similar methods of preparing the compounds are likewise known. Carboxylic acid derivatives of III may be employed in the above sequence with appropriate modifications in the reaction conditions. For example, an amide derivative of III may be substituted for the acid when condensing with the diamine II, preferably in the presence of a dehydrating agent or base at elevated temperatures, especially in the temperature range of 100-150°C. If E of Formula III is cyano, the reaction with the diamine II is typically performed in the presence of a catalytic quantity of an acid, such as p-toluenesulfonic acid, usually at temperatures of 120-180°C. If E is a thioamide derivative, the condensation with the diamine II is best effected in a solvent, such as ethylene glycol, at temperatures of 100-150°C. If, in Formula II, A is a halogen, the reaction is performed with the respective amidine derivative of III. The intermediate thus formed may be

first isolated or generated *in situ*, followed by cyclization at elevated temperatures, preferably in the range of 100-200°C.

In the preferred scheme above, when the acid III (E is -COOH) is unsubstituted or is substituted with unreactive functionalities (*e.g.*, alkyl, halogen, etc.), heating with the diamine II (A is amino) in polyphosphoric acid (PPA) is the most convenient and preferred method of preparing the respective imidazopyridine or purine. This method was described by Middleton and Wibberley, *J. Het. Chem.*, *17*, 1757 (1980), for the preparation of imidazo[4,5-b]- and [4,5-c]pyridines.

When the carboxylic acids of Formula III are substituted with groups such as alkoxy, PPA treatment can lead to dealkylation and the preferred conditions for the reaction are by refluxing the reactants in phosphorous oxychloride or xylene with the azeotropic removal of water.

Especially when the carboxylic acids (III) contain phenolic or amino substituents, an alternate method of preparation may be employed. A substituted aldehyde (III, E is -CHO) may be treated with sulfur and morpholine to produce the respective substituted-thioaryl carboxylic acid morpholide which on further treatment with methyl iodide gives the S-methyl-substituted-thioaryl carboxylic acid morpholide iodide derivative. Treatment of this intermediate with the appropriate diamine (II, A is amino) in a solvent such as ethylene glycol with heating at 40-150°C. produces the desired product I (Ia).

In accordance with another aspect of the invention, the compounds of Formula I (Ia) can be prepared by reacting a compound of the Formula IV

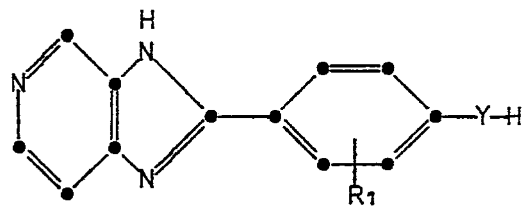

IV

or its tautomer, with a halo compound of the formula V

$$R_2\text{-G-X} \qquad V$$

where X is halo. The reaction is generally carried out by mixing approximately equimolar amounts of IV and V in a non-reactive solvent in the presence of an acid scavenger, although other ratios of IV and V are operative. Examples of non-reactive solvents include dioxane, dimethylsulfoxide, dimethylformamide, methylene chloride, toluene, and the like. Organic bases such as triethylamine or pyridine, or salts such as potassium carbonate may be employed as the acid scavenger. The reaction is carried out at temperatures from about 0°C. up to the reflux temperature of the reaction mixture, although temperatures of about 20-50°C. are preferred. This transformation can also be performed employing the analogous carboxylic or sulfonic anhydrides is place of the halides of Formula V.

The amino compounds ($R_2$=$-NR_3R_4$) may additionally be prepared by other methods known in the art. The most useful reaction is that of intermediate IV with phosgene in a non-reactive solvent to provide the corresponding chloroformic ester or isocyanate which upon treatment with the appropriate amine $HNR_3R_4$ provides the desired urea or carbamate I. The corresponding halo-sulfonamides and -sulfonic esters can also be prepared from IV according to standard procedures and then reacted with the appropriate amine to provide the corresponding sulfonamate ester or sulfamide derivative.

In addition, the above functionalizations of the phenol or aniline IV may be performed on related intermediates, i.e., compounds related to IV wherein the imidazole nitrogen atom is protected with a protecting group. As those skilled in the art will appreciate, such a protecting group would have to be stable and inert to the functionalization to provide an intermediate compound related to I where the nitrogen atom was similarly protected. Deprotecting this intermediate can then be accomplished to provide I. Compound protecting groups such as benzyl, which can be removed by hydrogenation, acetyl, which can be removed by mild hydrolysis, and the like may be employed.

The starting material 3,4-diaminopyridine is commercially available. Other required pyridines of Formula II are either commercially available, or may be prepared in the usual manner from available starting materials by the proper sequence of nitrations, reductions, acylations, hydrolyses, halogenations, and aminations. The required carboxylic acids and derivatives of Formula III are either commercially available, are known in the literature, or can be prepared by published methods as are the compounds of Formula V. Intermediates IV are taught and/or can be prepared by the methods described in European Patent Applications 72,926 and 79,083 and British Patent Application 2,119,377.

In addition, some of the compounds of Formula I (Ia) may be prepared by subsequent derivatizations of other compounds of Formula I (Ia) by methods known in the art. Thus, sulfide derivatives of Formula I (Ia) may be transformed into the respective sulfinyl and sulfonyl compounds, amine derivatives may be prepared from intermediate halo compounds, phenol substituents may be selectively alkylated, and like transformations. The sulfinyl and sulfonyl derivatives of this invention may be prepared directly by the reaction of the corresponding intermediates II with III, or by oxidation of the corresponding sulfide compounds of Formula I (Ia) by methods known in the art. One or two equivalents, respectively, of hydrogen peroxide in an alcohol, a peracid, such as meta-chloroperbenzoic acid in methylene chloride, or similar oxidants may be used to effect these transformations.

The pharmaceutically acceptable acid addition salts of this invention include salts derived from inorganic acids such as hydrochloric acid, nitric acid, phosphoric acid, sulfuric acid, hydrobromic acid, hydriodic acid, phosphorous acid and the like, as well as salts derived from nontoxic organic acids such as aliphatic mono- and di-carboxylic acids, phenyl-substituted alkanoic acids, hydroxy-alkanoic and -alkanedioic acids, aromatic acids, aliphatic and aromatic sulfonic acids, etc. The preferred salts of this invention are those derived from inorganic acids, especially hydrochloric acid.

The compounds may be administered by various routes including the oral, rectal, transdermal, subcutaneous, intravenous, intramuscular, or intranasal routes, being usually employed in the form of a pharmaceutical composition. It is a special feature of these compounds that they are effective positive inotropic agents, vasodilators, or bronchodilators following oral administration. Such compositions are prepared in a manner well known in the pharmaceutical art and comprise at least one active compound. Accordingly, the invention includes a pharmaceutical composition comprising as active ingredient a compound of Formula I (Ia) or an acid addition salt thereof, associated with a pharmaceutically acceptable carrier.

In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the composition can be in the form of tablets, pills, powders, lozenges, sachets, cachets, elixirs, suspensions, emulsions, solutions, syrups, aerosols (as a solid or in a liquid medium), ointments containing for example up to 10% by

weight of the active compound, soft and hard gelatin capsules, suppositories, sterile injectable solutions and sterile packaged powders.

Some examples of suitable carriers are lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propyl-hydroxybenzoates, talc, magnesium stearate or mineral oil. The formulations can additionally include lubricating agents, wetting agents, emulsifying and suspending agents, preserving agents, sweetening agents or flavoring agents. The compositions of the invention may, as is well known in the art, be formulated so as to provide quick, sustained or delayed release of the active ingredient after administration to the patient.

The compositions are preferably formulated in a unit dosage form, each dosage containing from about 5 to 500 mg., more usually 25 to 300 mg., of the active ingredient. The term "unit dosage form" refers to physically discrete units suitable as unitary dosages for human subjects and other mammals, each unit containing a predetermined quantity of active material calculated to produce the desired therapeutic effect, in association with the required pharmaceutical carrier.

The active compounds are effective over a wide dosage range and for example dosages per day will normally fall within the range of about 0.5 to 300 mg./kg. of body weight. In the treatment of adult humans, the range of from about 1 to 50 mg./kg., in

single or divided doses is preferred. However, it will be understood that the amount of the compound actually administered will be determined by a physician, in the light of the relevant circumstances including the condition to be treated, the choice of compound to be administered, the chosen route of administration, the age, weight, and response of the individual patient, and the severity of the patient's symptoms, and therefore the above dosage ranges are not intended to limit the scope of the invention in any way.

The following preparations and examples further illustrate the preparation of the compounds and formulations of this invention. The examples are illustrative only and are not intended to limit the scope of the invention in any way. The term "m/e" used in characterizing the products refers to the mass-to-charge ratio of ions which appear in the mass spectra of the products. In general, the values of the major peaks correspond to molecular weights, and are so designated "$M^+$".

## Example 1

2-(2-Methoxy-4-methylsulfonamidophenyl)imidazo[4,5-c]pyridine

A. Preparation of 2-(2-methoxy-4-nitrophenyl)-imidazo[4,5-c]pyridine.

A mixture of 1.1 g. of 3,4-diaminopyridine and 2.0 g. of 2-methoxy-4-nitrobenzoic acid in 100 ml. of

phosphorus oxychloride was heated at reflux for 16 hours. The reaction mixture was cooled to room temperature and the phosphorus oxychloride was removed *in vacuo*. The residue was dissolved in 300 ml. of 1N hydrochloric acid, taken to pH 8 with 50% sodium hydroxide and cooled to 10°C. The subtitle intermediate precipitated and was recovered by filtration to afford 1.5 g. of the compound as a yellow powder, m.p. 220-221.5°C.

Analysis: $C_{13}H_{10}N_4O_3$;

Calc.: C, 57.78; H, 3.73; N, 20.73;

Found: C, 57.79; H, 3.73; N, 20.73.

B. Preparation of 2-(4-amino-2-methoxyphenyl)imidazo[4,5-c]pyridine.

A mixture of 2.0 g. of 2-(2-methoxy-4-nitrophenylimidazo[4,5-c]pyridine and 0.2 g. of 5% palladium on carbon in 150 ml. of 95% ethanol and 50 ml. of tetrahydrofuran was hydrogenated at 60 psi for four hours. The catalyst was removed by filtration and the solvents were evaporated *in vacuo* giving 1.4 g. of the subtitle intermediate as a yellow foam. The NMR and mass spectra were consistent with the desired structure.

C. Preparation of 2-(2-methoxy-4-methylsulfonamidophenyl)imidazo[4,5-c]pyridine.

To a solution of 0.5 g. of 2-(4-amino-2-methoxyphenyl)imidazo[4,5-c]pyridine in 25 ml. of

tetrahydrofuran were added 0.6 ml. of triethylamine followed by 0.16 ml. of methanesulfonyl chloride. After stirring at room temperature for 20 minutes, the reaction mixture was evaporated in vacuo. The residue was chromatographed over silica gel. The appropriate fractions were combined and evaporated. Crystallization from isopropanol/diethyl ether provided 265 mg. of the title product, m.p. 63-66°C. The NMR and mass spectral data were consistent with the desired structure.

## Example 2

4-(Imidazo[4,5-c]pyridin-2-yl)-3-methoxyphenol, methanesulfonate (ester) hydrochloride

A. Preparation of 2-(2-methoxy-4-benzyloxyphenyl)imidazo[4,5-c]pyridine.

To a 60°C. solution of 7.0 g. of 4-benzyloxy-2-methoxybenzoic acid and 2.96 g. of 3,4-diaminopyridine in 250 ml. of pyridine were added 7.57 ml. of phosphorous oxychloride. The reaction was heated to 95°C. for 5 hours. After cooling to room temperature, the solvents were removed in vacuo. The residue was slurried in water and basified to pH 8 with 5N sodium hydroxide. The mixture was extracted with three 500 ml. portions of chloroform. The combined chloroform extracts were washed first with water and then with a saturated sodium chloride solution, dried over sodium sulfate, and evaporated to dryness. Chromatography over silica gel afforded 4.76 g. of the desired subtitle intermediate, m.p. 164-166°C.

Analysis: $C_{20}H_{17}N_3O_2$;
Calc.: C, 72.49; H, 5.17; N, 12.68;
Found: C, 72.23; H, 5.09; N, 12.46.

B. Preparation 2-(2-methoxy-4-hydroxyphenyl)-imidazo[4,5-c]pyridine.

A mixture of 4.46 g. of 2-(2-methoxy-4-benzyloxyphenyl)imidazo[4,5-c]pyridine and 1 g. of 5% palladium-on-carbon in 95 ml. of 3A ethanol was hydrogenated overnight at room temperature and 60 psi. Filtration of the reaction mixture and evaporation of the filtrate afforded 2.5 g. of the desired subtitle phenol which was used in the following reaction without additional purification. The NMR and mass spectral data were consistent with the assigned structure.

C. Preparation of 4-(imidazo[4,5-c]pyridin-2-yl)-3-methoxyphenol, methanesulfonate (ester) hydrochloride.

To a solution of 2-(2-methoxy-4-hydroxyphenyl)-imidazo[4,5-c]pyridine in 25 ml. of dimethylformamide were added 368 mg. of sodium hydride in portions. After hydrogen evolution had ceased, 0.71 ml. of methanesulfonyl chloride were added in a dropwise fashion. The reaction was stirred for 18 hours at room temperature and then diluted with 200 ml. of water. The resulting mixture was extracted with ethyl acetate. The organic extract was washed with water and a saturated sodium chloride solution, dried over sodium sulfate, and

evaporated to an oil. The aqueous layer from above was concentrated *in vacuo* and combined with the oil for chromatography over silica gel. The appropriate fractions were combined and evaporated to provide an oily residue. The residue was crystallized from dimethylformamide containing 1.5 equivalents of concentrated hydrochloric acid to provide 1.92 g. of the title product, m.p. 215-216°C. with decomposition.

Analysis: $C_{14}H_{14}ClN_3O_4S$;
Calc.: C, 47.26; H, 3.97; N, 11.81; Cl, 9.96;
Found: C, 47.06; H, 4.13; N, 11.63; Cl, 10.06.

## Example 3

Hard gelatin capsules are prepared using the following ingredients:

| | Quantity (mg./capsule) |
|---|---|
| Active compound | 250 |
| Starch dried | 200 |
| Magnesium stearate | 10 |

The above ingredients are mixed and filled into hard gelatin capsules in 460 mg. quantities.

## Example 4

A tablet formula is prepared using the ingredients below:

| | Quantity (mg./tablet) |
|---|---|
| Active compound | 250 |
| Cellulose, microcrystalline | 400 |
| Silicon dioxide, fumed | 10 |
| Stearic acid | 5 |

The components are blended and compressed to form tablets each weighing 665 mg.

## Example 5

An aerosol solution is prepared containing the following components:

| | Weight % |
|---|---|
| Active ingredient | 0.25 |
| Ethanol | 29.75 |
| Propellant 22 (Chlorodifluoromethane) | 70 |

The active compound is mixed with ethanol and the mixture added to a portion of the propellant 22, cooled to -30°C. and transferred to a filling device. The required amount is then fed to a stainless steel container and diluted with the remaining amount of propellant. The valve units are then fitted to the container.

Example 6

Tablets each containing 60 mg. of active ingredient are made up as follows:

| | |
|---|---|
| Active ingredient | 60 mg. |
| Starch | 45 mg. |
| Microcrystalline cellulose | 35 mg. |
| Polyvinylpyrrolidone (as 10% solution in water) | 4 mg. |
| Sodium carboxymethyl starch | 4.5 mg. |
| Magnesium stearate | 0.5 mg. |
| Talc | 1 mg. |
| | -- |
| Total | 150 mg. |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60°C. and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Example 7

Capsules each containing 80 mg. of medicament are made as follows:

| | |
|---|---|
| Active ingredient | 80 mg. |
| Starch | 59 mg. |
| Microcrystalline cellulose | 59 mg. |
| Magnesium stearate | 2 mg. |
| Total | 200 mg. |

The active ingredient, cellulose, starch and magnesium stearate are blended, passed through a No. 45 mesh U.S. sieve, and filled into hard gelatin capsules in 200 mg. quantities.

Example 8

Suppositories each containing 225 mg. of active ingredient are made as follows:

| | |
|---|---|
| Active ingredient | 225 mg. |
| Saturated fatty acid glycerides to | 2,000 mg. |

The active ingredient is passed through a No. 60 mesh U.S. sieve and suspended in the saturated fatty acid glycerides previously melted using the minimum heat necessary. The mixture is then poured into a suppository mold of nominal 2 g. capacity and allowed to cool.

## Example 9

Suspensions each containing 50 mg. of medicament per 5 ml. dose are made as follows:

| | |
|---|---|
| Active ingredient | 50 mg. |
| Sodium carboxymethyl cellulose | 50 mg. |
| Syrup | 1.25 ml. |
| Benzoic acid solution | 0.10 ml. |
| Flavor | q.v. |
| Color | q.v. |
| Purified water to | 5 ml. |

The medicament is passed through a No. 45 mesh U.S. sieve and mixed with the sodium carboxymethylcellulose and syrup to form a smooth paste. The benzoic acid solution, flavor and color are diluted with some of the water and added, with stirring. Sufficient water is then added to produce the required volume.

The compounds of this invention and their pharmaceutically acceptable salts have been found to possess useful pharmaceutical properties, including positive inotropy, vasodilation, and anticoagulation. In addition, the compounds have phosphodiesterase inhibition activity and are active in the Herxheimer assay, suggesting their use as compounds effective in treating or preventing bronchial asthma, chronic obstructive pulmonary disease, infant apnea, and related disorders. Certain compounds of the present invention were examined as to their pharmacodynamic effects in the following test systems.

Positive Inotropic Activity in Isolated Cat Papillary Muscles

Cats of either sex were anesthetized with Metofane (1,1-difluoro-2,2-dichloroethyl methyl ether, Pittman-Moore) their hearts immediately removed and the papillary muscles dissected and suspended in individual organ baths. A platinum hook secured one end of the muscle to an electrode mounted in the bottom of the bath, and a silk thread attached the tendon to a Statham isometric transducer. The baths contained Krebs-Henseleit solution (36°C., bubbled with 95 percent oxygen - 5 percent carbon dioxide) of the following millimolar composition: NaCl, 118; KCl, 4.5; $CaCl_2$, 2.5; $KH_2PO_4$, 1.1; $MgSO_4$, 1.2; $NaHCO_3$, 25; and glucose, 11.

A base-line tension of 1.5 g. was applied to each muscle. Square-wave pulses (5.0 msec. in duration, 20 percent above threshold voltage) delivered through the hook electrode and a second electrode positioned near the top of the muscle evoked 12 contractions/minute, which were recorded on a Grass polygraph. After the muscles were equilibrated for 60 minutes, drugs were introduced in normal saline to bring the final concentration of the drug to $10^{-5}$ or $10^{-4}$ molar. Increases in contractility were tabulated as millimeters of pen deflection in excess of the baseline values. In each experiment the maximum contractility was measured. As a percent of control (control = 100 percent), the compound of Example 1 produced a contractility of 149% at $10^{-5}$ M and 168% at $10^{-4}$ M and the compound of Example 2 gave a contractility of 240% at $10^{-5}$M and 326% at $10^{-4}$M.

Experiments in Anesthetized Dogs

Mongrel dogs of either sex ranging in weight from 7 to 14 kg. were used. Anesthesia was induced with sodium pentobarbital (30 mg./kg., i.v.) and maintained with supplemental doses as required. A positive-pressure pump was used to ventilate the dogs through an endotracheal tube (18 strokes/minute, 20 ml./kg. $stroke^{-1}$), and a heating pad kept the body temperature at 37-38°C.

Femoral arterial blood pressure was measured through a polyethylene catheter filled with heparin solution (16 units/ml.) and connected to a Statham pressure transducer. A strain-gauge arch sutured to the right ventricle of the heart measured cardiac contractility. Tension on the gauge was adjusted to 50 g. and the gain of the recorder (Beckman dynograph) was set so that 50 g. caused a 10-mm. pen deflection; cardiac contractile tension was measured as millimeters of pen deflection or grams of tension. The drug was administered following a 30-45 minute equilibrium period as an i.v. bolus (2-5 ml.) in a normal saline vehicle. In a control experiment, rapid intravenous injection of 50 ml. of 5 percent dextran and mechanical compression of the aorta showed that the contractility measurements were independent of changes in preload and afterload. Heart rate was derived by means of a cardiotach which was triggered by the arterial pressure pulse signal and displayed on the polygraph. The maximum effects on contractility at various dose levels were calculated as a percent of control (control = 100 percent). The

compound of Example 1 produced an $ED_{50}$ (dose, i.v., which produced a peak increase of 50% over control) of 0.030 mg./kg. while the compound of Example 2 gave an $ED_{50}$ of 6 mcg./kg.

## CLAIMS

1. A compound of the formulas

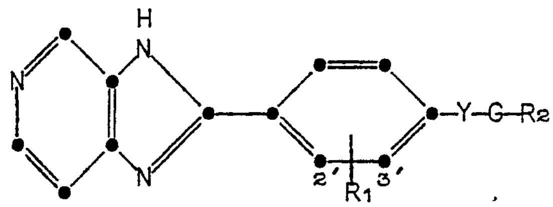

, I

and

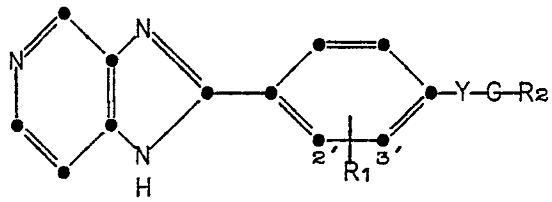

Ia

or a pharmaceutically acceptable salt thereof, wherein:

$R_1$ is hydrogen, $C_1$-$C_4$ alkyl, $C_1$-$C_4$ alkoxy, allyloxy, propargyloxy, benzyloxy, ($C_1$-$C_4$ alkyl)thio, ($C_1$-$C_4$ alkyl)sulfinyl, ($C_1$-$C_4$ alkyl)sulfonyl, hydroxy, halo, cyano, nitro, amino, mono- or di-($C_1$-$C_4$ alkyl)-amino, trifluoromethyl or Z-Q-substituted $C_1$-$C_4$ alkoxy, wherein Q is oxygen, sulfur, sulfinyl, sulfonyl, or a bond, and Z is $C_1$-$C_4$ alkyl, phenyl or phenyl substituted with halo, $C_1$-$C_4$ alkyl, $C_1$-$C_4$ alkoxy, hydroxy, nitro, amino, ($C_1$-$C_4$ alkyl)thio, ($C_1$-$C_4$ alkyl)sulfinyl, or ($C_1$-$C_4$ alkyl)sulfonyl;

Y is -O- or -NH-;

G is C=O or $-S(O)_r-$, where r is 0, 1, or 2;

and

$R_2$ is $C_1$-$C_4$ alkyl, phenyl, trifluoromethyl, or $-NR_3R_4$,
where each of $R_3$ and $R_4$ is independently hydrogen or $C_1$-$C_3$ alkyl; with the limitation that when $R_2$ is trifluoromethyl or $-NR_3R_4$, and G is $-S(O)_r-$, r may not be 0 or 1, and with the further limitation that when Y is -O- and G is $-S(O)_r-$ may not be 0 or 1.

2. The compound of claim 1 which is 2-(2-methoxy-4-methanesulfonamidophenyl)imidazo[4,5-c]pyridine or a pharmaceutically acceptable salt thereof.

3. The compound of claim 1 which is 4-(imidazo[4,5-c]pyridin-2-yl)-3-methoxyphenol, methane sulfonate (ester) or a pharmaceutically acceptable salt thereof.

4. A process for preparing a compound of the formulas

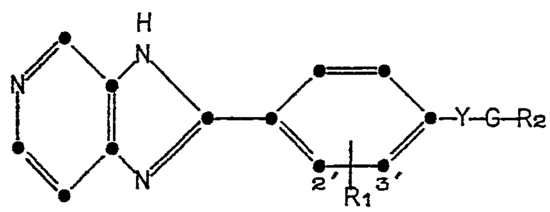

I

and

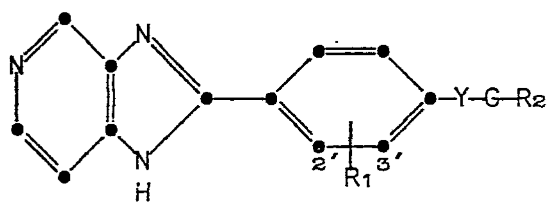

Ia

or a pharmaceutically acceptable salt thereof, wherein:

$R_1$ is hydrogen, $C_1$-$C_4$ alkyl, $C_1$-$C_4$ alkoxy, allyloxy, propargyloxy, benzyloxy, ($C_1$-$C_4$ alkyl)thio, ($C_1$-$C_4$ alkyl)sulfinyl, ($C_1$-$C_4$ alkyl)sulfonyl, hydroxy, halo, cyano, nitro, amino, mono- or di-($C_1$-$C_4$ alkyl)-amino, trifluoromethyl or Z-Q-substituted $C_1$-$C_4$ alkoxy, wherein Q is oxygen, sulfur, sulfinyl, sulfonyl, or a bond, and Z is $C_1$-$C_4$ alkyl, phenyl or phenyl substituted with halo, $C_1$-$C_4$ alkyl, $C_1$-$C_4$ alkoxy, hydroxy, nitro, amino, ($C_1$-$C_4$ alkyl)thio, ($C_1$-$C_4$ alkyl)sulfinyl, or ($C_1$-$C_4$ alkyl)sulfonyl;

Y is -O- or -NH-;

G is C=O or $-S(O)_r-$, where r is 0, 1, or 2; and

$R_2$ is $C_1$-$C_4$ alkyl, phenyl, trifluoromethyl, or $-NR_3R_4$, where each of $R_3$ and $R_4$ is independently hydrogen or $C_1$-$C_3$ alkyl; with the limitation that when $R_2$ is trifluoromethyl or $-NR_3R_4$, and G is $-S(O)_r-$, r may not be 0 or 1, and with the further limitation that when Y is -O- and G is $-S(O)_r-$ may not be 0 or 1, which comprises reacting a compound of the Formula IV

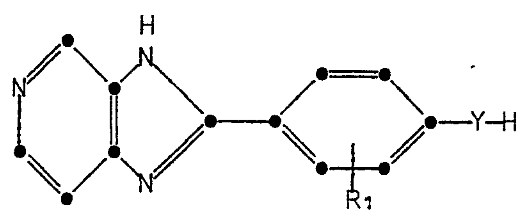

IV

or its tautomer, wherein $R_1$ and Y are as defined above, with a halo compound of the formula V

$$R_2\text{-G-X} \qquad V$$

wherein G is as defined above and X is halo.

5. A compound of claim 1 for use in producing a positive inotropic effect, delaying coagulation of the blood, causing bronchodilation, or causing vasodilation in a mammal.

6. A pharmaceutical composition which comprises a compound of claim 1 and a pharmaceutically acceptable carrier or diluent.

7. A composition of claim 5 wherein $R_1$ is methoxy.

8. A composition of claim 5 or 6 wherein G is $-S(O)_r-$.

9. A composition of claim 5 wherein the compound is 2-(2-methoxy-4-methanesulfonamidophenyl)-imidazo[4,5-c]pyridine or a pharmaceutically acceptable salt thereof.

10. A composition of claim 5 wherein the compound is 4-(imidazo[4,5-c]pyridin-2-yl)-3-methoxyphenol, methanesulfonate (ester) or a pharmaceutically acceptable salt thereof.

## CLAIMS

1. A process for preparing compound of the formulas

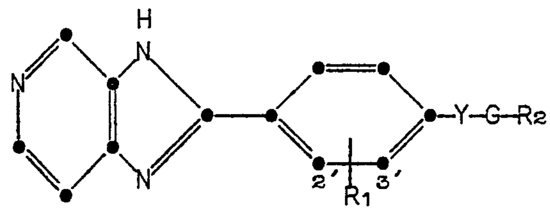

I

and

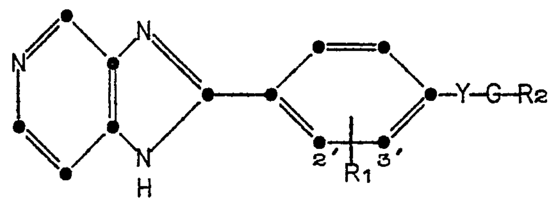

Ia

or a pharmaceutically acceptable salt thereof, wherein:

$R_1$ is hydrogen, $C_1$-$C_4$ alkyl, $C_1$-$C_4$ alkoxy, allyloxy, propargyloxy, benzyloxy, ($C_1$-$C_4$ alkyl)thio, ($C_1$-$C_4$ alkyl)sulfinyl, ($C_1$-$C_4$ alkyl)sulfonyl, hydroxy, halo, cyano, nitro, amino, mono- or di-($C_1$-$C_4$ alkyl)-amino, trifluoromethyl or Z-Q-substituted $C_1$-$C_4$ alkoxy, wherein Q is oxygen, sulfur, sulfinyl, sulfonyl, or a bond, and Z is $C_1$-$C_4$ alkyl, phenyl or phenyl substituted with halo, $C_1$-$C_4$ alkyl, $C_1$-$C_4$ alkoxy, hydroxy, nitro, amino, ($C_1$-$C_4$ alkyl)thio, ($C_1$-$C_4$ alkyl)sulfinyl, or ($C_1$-$C_4$ alkyl)sulfonyl;

Y is -O- or -NH-;

G is C=O or $-S(O)_r-$, where r is 0, 1, or 2;

and

$R_2$ is $C_1$-$C_4$ alkyl, phenyl, trifluoromethyl, or $-NR_3R_4$, where each of $R_3$ and $R_4$ is independently hydrogen or $C_1$-$C_3$ alkyl; with the limitation that when $R_2$ is trifluoromethyl or $-NR_3R_4$, and G is $-S(O)_r-$, r may not be 0 or 1, and with the further limitation that when Y is -O- and G is $-S(O)_r-$ may not be 0 or 1, which comprises reacting a compound of the Formula IV

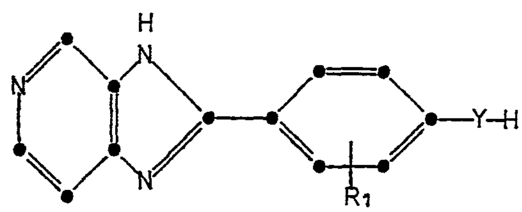

IV

or its tautomer, wherein $R_1$ and Y are as defined above, with a halo compound of the formula V

$R_2$-G-X V

wherein G is as defined above and X is halo.

2. The process of claim 1 wherein $R_1$ is methoxy.

3. The process of claim 2 wherein $-GR_2$ is methane sulfonyl.

4. The process of claim 3 wherein Y is -O-.

5. The process of claim 3 wherein Y is -NH-.

6. A compound of formula I or Ia whenever produced by the process of any one of claims 1 to 5.

0155094

European Patent Office

# EUROPEAN SEARCH REPORT

Application number

EP 85 30 1114

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 098 448 (DR. K. THOMMAE GMBH) * Page 1, lines 9-16; page 5, line 5 - page 7, line 14; page 10, line 19 - page 11, line 7; page 17, line 8 - page 21, line 21; compounds T,U; examples 14,16 * --- | 1-10 | C 07 D 471/04<br>A 61 K 31/435//<br>(C 07 D 471/04<br>C 07 D 235:00<br>C 07 D 221:00 ) |
| X,D | EP-A-0 072 926 (MERCK PATENT GMBH) * Page 14, line 24 - page 16, line 27; example 13 in combination with the compounds on page 30, lines 2-3,4,16,34-35, page 35, line 13, page 36, lines 16-17 * --- | 1 | |
| A | EP-A-0 093 593 (ELI LILLY AND CO.) * Page 1,line 1 - page 2, line 10; page 20, line 9 - page 23, line 22 * & GB-A-2 119 377 (Cat. D) --- | 1,5,6 | TECHNICAL FIELDS SEARCHED (Int. Cl.4)<br><br>C 07 D 471/00<br>A 61 K 31/00 |
| A,D | EP-A-0 079 083 (THE WELLCOME FOUNDATION LTD.) * Page 1, line 20 - page 2, line 11; page 4, line 5 - page 5, line 8; page 6, line 22 - page 8, line 19 * ----- | 1,5,6 | |

The present search report has been drawn up for all claims

| Place of search | Date of completion of the search | Examiner |
|---|---|---|
| BERLIN | 07-05-1985 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document